# EUROPÄISCHE PATENTSCHRIFT

(11) **EP 3 823 717 B1**
(45) Veröffentlichungstag und Bekanntmachung des Hinweises auf die Patenterteilung: **07.06.2023**
(21) Anmeldenummer: 19744647.9
(22) Anmeldetag: 17.07.2019
(51) Int. Cl.: A61N 1/365

(54) **HERZSCHRITTMACHERZELLEN AUF CHIP ZUR ETABLIERUNG EINES AUTONOM REGULIERBAREN ELEKTRISCHEN SCHRITTMACHERS**
ON-CHIP PACEMAKER CELLS FOR ESTABLISHING AN AUTONOMOUSLY CONTROLLABLE ELECTRICAL PACEMAKER
CELLULES DE STIMULATEUR CARDIAQUE SUR PUCE POUR L'ÉTABLISSEMENT D'UN STIMULATEUR CARDIAQUE ÉLECTRIQUE RÉGLABLE DE FAÇON AUTONOME

(30) Priorität: 18.07.2018 DE 102018212005
(43) Veröffentlichungstag der Anmeldung: 26.05.2021
(73) Patentinhaber: Universität Rostock, 18055 Rostock (DE)
(72) Erfinder: DAVID, Robert, 18209 Wittenbeck (DE); RIMMBACH, Christian, 18107 Lichtenhagen (DE); JUNG, Julia, 18107 Lichtenhagen (DE)
(74) Vertreter: Altmann Stößel Dick Patentanwälte PartG mbB
(86) Internationale Anmeldenummer: PCT/EP2019/069196
(87) Internationale Veröffentlichungsnummer: WO 2020/016273

(56) Entgegenhaltungen:
- US-A1- 2006 134 071
- US-A1- 2009 053 180
- US-A1- 2013 330 378

## Beschreibung

Die vorliegende Erfindung betrifft ein bioelektronisches System, umfassend mindestens i) *in vitro* generiertes Herzschrittmachergewebe umfassend Herz-Schrittmacherzellen; ii) eine auf einem Halbleiter-Träger aufgebrachte elektronische Schaltung umfassend mindestens einen Feldeffekttransistor; wobei das *in vitro* generierte Herzschrittmachergewebe umfassend Herz-Schrittmacherzellen gemäß i) auf der auf dem Halbleiter-Träger aufgebrachten elektronischen Schaltung gemäß ii) immobilisiert und in leitfähiger Kommunikation zu dem mindestens einen Feldeffekttransistor angeordnet sind. Weiterhin betrifft die Erfindung ein frequenzadaptives Herzschrittmachersystem umfassend mindestens einen frequenzadaptiven Herzschrittmacher, umfassend mindestens eine Sensoreinheit, wobei die Sensoreinheit eingerichtet ist, um mindestens ein elektrisches Herzsignal zu detektieren; mindestens einen Pulsgenerator, wobei der Pulsgenerator eingerichtet ist, um mindestens einen elektrischen Schrittmacherimpuls zu erzeugen und dem Herz eines Patienten zuzuführen; mindestens eine Steuereinheit, wobei die Steuereinheit elektrisch verbunden ist mit der Sensoreinheit und dem Pulsgenerator; ein bioelektronisches System, wobei das bioelektronische System leitfähig mit dem frequenzadaptiven Herzschrittmacher verbunden oder verbindbar ist, insbesondere mit der Steuereinheit.

Ein Herzschrittmacher ist ein elektronischer Impulsgenerator, der zur elektrischen Anregung des Herzmuskels zur Kontraktion eingesetzt wird, um Herzrhythmusstörungen auszugleichen. Natürliche Herzschrittmacher sind der Sinusknoten und teilweise auch der Atrioventrikularknoten. Künstliche Herzschrittmacher sind elektronische Geräte, welche zumeist implantiert werden und welche ebenfalls als elektronische Impulsgeneratoren fungieren. Im Gegensatz zum natürlichen Schrittmacher Sinusknoten sprechen derzeit elektronische Herzschrittmacher nicht auf autonome Stimulation wie beispielsweise körperliche Belastung oder psychische Erregung an, die Herzfrequenz wird daher von elektronischen Herzschrittmachern nicht an diese physiologischen Zustände des Körpers angepasst.

Die funktionelle Ankoppelung von Nervenzellen an Halbleiterchips ist prinzipiell bekannt und wird beispielsweise von Fromherz im Forschungsbericht 2009 des Max-Planck-Instituts für Biochemie beschrieben. Ebenso sind diverse Ansätze für die Erzeugung von Herzschrittmacherzellen bekannt, beispielsweise über forward-Programmierung aus multipotenten oder pluripotenten Stammzellen (siehe WO 2015/091157 A1, WO 2017/108895 A1), mittels direkter Programmierung aus embryonalen Stammzellen, oder mittels Umprogrammierung aus beispielsweise Kardiomyozyten (WO 2013/070952 A1) bekannt. Die US 2009/053180 A1 beschreibt ein Tandem-Schrittmachersystem, umfassend (1) einen elektronischen Schrittmacher und (2) einen biologischen Schrittmacher, wobei der biologische Schrittmacher eine implantierbare Zelle umfasst, die einen hyperpolarisationsaktivierten, zyklischen Nukleotidgesteuerten (HCN)-Ionenkanal funktionell exprimiert, und wobei das exprimierte Der HCN-Kanal erzeugt einen wirksamen Schrittmacherstrom, wenn die Zelle in das Herz eines Patienten implantiert wird. US 2003/0036773 A1 beschreibt ein bioelektronisches Herzschrittmachersystem mit einem Feldeffekttransistor.

Eine der vorliegenden Erfindung zugrunde liegende Aufgabe bestand daher darin, einen Herzschrittmacher bzw. hierfür geeignete Komponenten bereitzustellen, welche(r) in der Lage sein sollte(n), die wesentlichen Nachteile der konventionellen elektronischen Schrittmacher zu beheben.

Überraschend wurde gefunden, dass mit einem bioelektronisches System ein solcher Herzschrittmacher bzw. dessen Komponenten bereitgestellt werden können, in dem *in vitro* generiertes Herzschrittmachergewebe umfassend Herz-Schrittmacherzellen auf einer auf einem Halbleiter-Träger aufgebrachten elektronischen Schaltung immobilisiert und in leitfähiger Kommunikation zu mindestens einem Feldeffekttransistor angeordnet werden.

Die Erfindung betrifft daher ein bioelektronisches System, umfassend mindestens
i) *in vitro* generiertes Herzschrittmachergewebe umfassend Herz-Schrittmacherzellen;
ii) eine auf einem Halbleiter-Träger aufgebrachte elektronische Schaltung umfassend mindestens einen Feldeffekttransistor;
wobei das *in vitro* generierte Herzschrittmachergewebe umfassend Herz-Schrittmacherzellen gemäß i) auf der auf dem Halbleiter-Träger aufgebrachten elektronischen Schaltung gemäß ii) immobilisiert und in leitfähiger Kommunikation zu dem mindestens einen Feldeffekttransistor angeordnet ist.

Unter einem Feldeffekttransistor (FET) wird im Rahmen der vorliegenden Erfindung grundsätzlich eine Funktionseinheit verstanden, welche mindestens eine Source-Elektrode, mindestens eine Drain-Elektrode und mindestens eine Gate-Elektrode umfasst. Weiterhin umfasst der Feldeffekttransistor mindestens einen Source-Drain-Kanal, wobei durch den Source-Drain-Kanal ein Strom zwischen der Source-Elektrode und der Drain-Elektrode fließen kann, zumindest unter spezifischen äußeren Bedingungen. Diese Bedingungen können insbesondere eine an dem Source-Drain-Kanal anliegende Spannung oder ein an dem Source-Drain-Kanal anliegendes elektrisches Potential umfassen. Hierbei kann das elektrische Potential insbesondere mittels der weiter unten noch näher beschriebenen Gate-Elektrode oder mittels einer externen Elektrode an den Source-Drain-Kanal angelegt sein. Der Source-Drain-Kanal kann mindestens ein Halbleitermaterial umfassen, insbesondere ein dotiertes Halbleitermaterial. Die Gate-Elektrode kann mindestens eine flüssige Lösung umfassen, wobei die Lösung mindestens einen Elektrolyten umfassen kann. Ein FET umfassend eine solche Gate-Elektrode kann auch als "liquid-gated FET" bezeichnet werden. Weiterhin kann der Source-Drain-Kanal eine isolierende Schicht, insbesondere umfassend mindestens eine Oxidschicht, aufweisen, wobei die isolierende Schicht den Source-Drain-Kanal zumindest teilweise von der Gate-Elektrode elektrisch isolieren kann.

Gemäß einer bevorzugten Ausführungsform des bioelektronisches Systems ist das *in vitro* generierte Herzschrittmachergewebe umfassend Herz-Schrittmacherzellen aus Myokardzellen, bevorzugt mittels Umprogrammierung, erzeugt. Die direkte Umprogrammierung kann beispielsweise aus ventrikulären Kardiomyozyten, ggf. unter Verwendung von TBX-Faktoren wie beispielsweise TBX18, erfolgen. Exemplarisch ist eine entsprechende Umprogrammierung in Beispiel 1 der WO 2013/070952 A1 offenbart, deren Offenbarung hier durch Bezugnahme integriert ist.

In einer Ausführungsform des bioelektronisches Systems ist das *in vitro* generierte Herzschrittmachergewebe umfassend Herz-Schrittmacherzellen aus embryonalen Stammzellen, bevorzugt mittels direkter Programmierung, weiter bevorzugt mittels direkter Programmierung unter Einsatz von Shox2, erzeugt. Eine entsprechende direkte Programmierung ist beispielsweise in Beispiel 4 der WO 2013/070952 A1 offenbart, welches hier durch Bezugnahme integriert ist.

In einer Ausführungsform des bioelektronisches Systems ist das *in vitro* generierte Herzschrittmachergewebe umfassend Herz-Schrittmacherzellen aus multipotenten oder pluripotenten Stammzellen, bevorzugt aus pluripotenten Stammzellen, bevorzugt mittels forward-Programmierung, erzeugt (induzierte sinuatriale Zellkörperchen (iSABs) umfassend Herz-Schrittmacherzellen). Bevorzugt werden hierbei Sinusknotenzellen (Herz-Schrittmacherzellen) aus Stammzellen erzeugt, bei welchem eine Nukleinsäure in Stammzellen eingebracht wird, wodurch diese einen TBX-Transkriptionsfaktor exprimieren, oder ein TBX-Protein in die Stammzellen eingebracht wird, wobei zusätzlich ein Konstrukt zur Expression eines Antibiotikum-Resistenz-Gens, welches durch einen alpha-MHC (MYH6)-Promoter gesteuert wird, eingebracht wird und die resultierenden Stammzellen in Gegenwart des Antibiotikums differenziert werden. Beschrieben ist diese Erzeugung von Sinusknotenzellen in der WO 2015/091157 A1, deren Offenbarung hier durch Bezugnahme aufgenommen ist. Insbesondere ist in dieser Ausführungsform das *in vitro* generierte Herzschrittmachergewebe umfassend Herz-Schrittmacherzellen aus multipotenten oder pluripotenten Stammzellen, bevorzugt aus pluripotenten Stammzellen, erzeugt, wobei mindestens ein TBX-Transkriptionsfaktor, insbesondere TBX3, in Kombination mit einer Antibiotikaselektion auf der Basis des Myh6-Promotors eingesetzt wird.

Wenn eine Nukleinsäure zur Expression eines TBX-Transkriptionsfaktors in die Stammzellen eingebracht wird, wird diese vorzugsweise ausgewählt aus TBX-DNS, insbesondere TBX-cDNS; oder TBX-RNS, insbesondere TBX-mRNS. Im Rahmen der RNS können TBX-mRNS in die Stammzellen transfiziert werden, wobei dies keine stabile Gen-Modifikation ergibt. Alternativ können mikro-RNS eingebracht werden, die endogenes TBX zur Expression bringen. In einer bevorzugten Ausführungsform der Nukleinsäure-Einbringung wird TBX-DNS, insbesondere TBX-cDNS mittels Vektor, insbesondere mittels (Über-)Expressionsvektor, eingebracht. Das TBX ist vorzugsweise ausgewählt aus TBX3 oder TBX-18, wobei TBX3 besonders bevorzugt und TBX3-cDNS höchst bevorzugt ist. D.h. eine höchst bevorzugte Variante ist die Einbringung von TBX3-cDNS mit Überexpressionsvektor. Hinsichtlich des TBX-Proteins, welches ebenfalls keine (stabile) Gen-Modifikation verursacht, ist ebenfalls TBX3 bevorzugt. Eingesetzt werden humane oder nicht-humane Nukleinsäuren oder Proteine, wobei diejenigen humaner Herkunft bevorzugt sind.

Hinsichtlich der eingesetzten Stammzellen werden multipotente oder pluripotente, vorzugsweise pluripotente, Stammzellen eingesetzt. Die Stammzellen können ausgewählt werden aus humanen oder nicht-humanen embryonalen Stammzellen oder humanen oder nicht-humanen induzierten Stammzellen oder humanen induzierten Stammzellen oder parthenogenetischen Stammzellen oder spermatogonialen Stammzellen. Bevorzugt ist das Herzschrittmachergewebe umfassend Herz-Schrittmacherzellen aus nicht-humanen embryonalen Stammzellen oder nicht-humanen induzierten pluripotenten Stammzellen oder humanen induzierten pluripotenten Stammzellen oder parthenogenetischen Stammzellen oder spermatogonialen Stammzellen erzeugt, bevorzugt mittels forward-Programmierung, besonders bevorzugt aus nicht-humanen embryonalen Stammzellen oder nicht-humanen induzierten pluripotenten Stammzellen oder humanen induzierten pluripotenten Stammzellen. Humane embryonale Stammzellen sind in dieser bevorzugten sowie besonders bevorzugten Variante explizit ausgenommen.

Die Antibiotikaselektion auf der Basis des Myh6-Promotors nutzt vorzugsweise ein Antibiotikum-Resistenz-Gen ausgewählt aus Aminoglykosid-Antibiotikum-Resistenz-Gen, mehr bevorzugt aus Neomycin- und Puromycin-Resistenz-Gen, höchst bevorzugt Neomycin-Resistenz-Gen. Das für die Selektion eingesetzte Antibiotikum ist entsprechend ausgewählt aus Aminoglykosid-Antibiotikum, insbesondere aus Neomycin und Puromycin. "Entsprechend ausgewählt" bedeutet, dass immer das zum Resistenz-Gen passende Antibiotikum eingesetzt wird, beispielsweise beim Neomycin-Resistenz-Gen wird anschließend mit Neomycin selektiert. Erzeugt und entsprechend eingesetzt werden in jedem Fall Herz-Schrittmacherzellen (human oder nicht human), wobei humane Herz-Schrittmacherzellen bevorzugt sind. Hierfür werden humane Stammzellen mit vorzugsweise humanem Protein oder humaner Nukleinsäure kombiniert. Kreuzkombinationen, wie beispielsweise die Einführung humaner Proteine oder humaner Nukleinsäure in nicht-humane, z.B. murine, Stammzellen ist ebenfalls möglich, auch die reine Kombination der nicht-humanen Vertreter zur Erzeugung nicht humaner Herz-Schrittmacherzellen.

Das *in vitro* generierte Herzschrittmachergewebe umfassend Herz-Schrittmacherzellen steht bevorzugt zumindest teilweise in elektrischem Kontakt mit einer Gate-Elektrode des Feldeffekttransistors.

Bevorzugt umfasst die Gate-Elektrode mindestens einen Elektrolyten, insbesondere besteht die Gate-Elektrode aus einer Lösung umfassend den mindestens einen Elektrolyten.

In einer bevorzugten Ausführungsform des bioelektronischen Systems ist ein an der Gate-Elektrode anliegendes elektrisches Potential direkt oder indirekt durch ein von dem *in vitro* generierten Herzschrittmachergewebe umfassend Herz-Schrittmacherzellen erzeugtes elektrisches Primärsignal beeinflussbar. Unter einem "elektrischen Primärsignal" wird im Rahmen der vorliegenden Erfindung grundsätzlich ein beliebiges elektrisches Signal verstanden, welches aufgrund eines vorausgegangenen physiologischen Ereignisses und/oder aufgrund mindestens eines eintreffenden physiologischen Signals, insbesondere eines Moleküls, beispielsweise eines Hormons, erzeugt wird. Das von dem *in vitro* generierten Herzschrittmachergewebe umfassend Herz-Schrittmacherzellen erzeugte elektrische Primärsignal umfasst mindestens einen lonenstrom, insbesondere einen lonenstrom umfassend Kalium-Ionen und/oder Calcium-Ionen. So kann der von dem *in vitro* generierten Herzschrittmachergewebe umfassend Herz-Schrittmacherzellen erzeugte lonenstrom in die Lösung der Gate-Elektrode strömen und die Ladungsverhältnisse der Lösung der Gate-Elektrode und damit das an dem Source-Drain-Kanal anliegende elektrische Potential oder die an dem Source-Drain-Kanal anliegende Spannung verändern. Insbesondere kann der lonenstrom das an dem Source-Drain-Kanal anliegende elektrische Potential oder die an dem Source-Drain-Kanal anliegende Spannung derart verändern, dass der Source-Drain-Kanal elektrisch leitend wird für einen Strom zwischen der Source-Elektrode und der Drain-Elektrode, wie weiter unten noch näher beschrieben.

In einer bevorzugten Ausführungsform des bioelektronischen Systems ist das von dem *in vitro* generierten Herzschrittmachergewebe umfassend Herz-Schrittmacherzellen erzeugte elektrische Primärsignal durch einen physiologischen Faktor einer Umgebung des in vitro generierten Herzschrittmachergewebes umfassend Herz-Schrittmacherzellen beeinflussbar. Insbesondere kann es möglich sein, dass ein physiologischer Faktor eine Erzeugung des elektrischen Primärsignals verursachen oder unterdrücken kann oder eine Eigenschaft des elektrischen Primärsignals, wie beispielsweise eine Signalstärke oder eine Signalfrequenz einer Abfolge von elektrischen Primärsignalen, beeinflussen, insbesondere bestimmen, kann. Ein positiv chronotroper Effekt (Frequenzsteigerung) kann über Stimulation des Sympathikus durch die Transmitter Adrenalin und Noradrenalin erzielt werden, die über die Blutbahn zu den Zielzellen gelangen. Ein negativ chronotroper Effekt (Frequenzerniedrigung) ergibt sich durch parasympathische Stimulation mittels Acetylcholin über den Nervus vagus.

In einer Ausführungsform des bioelektronischen Systems steht die Gate-Elektrode in direktem oder indirektem physischen Kontakt mit mindestens einem Source-Drain-Kanal des Feldeffekttransistors. So kann die Gate-Elektrode insbesondere durch die isolierende Schicht, insbesondere die Oxidschicht, von dem Source-Drain-Kanal getrennt sein. Die die Gate-Elektrode kann jedoch auch in direktem physischen Kontakt zu dem Source-Drain-Kanal stehen.

Bevorzugt ist in dem Source-Drain-Kanal durch das elektrische Primärsignal mittels einer Beeinflussung des an der Gate-Elektrode anliegenden elektrischen Potentials ein elektrisches Antwortsignal erzeugbar. Unter einem "elektrischen Antwortsignal" wird im Rahmen der vorliegenden Erfindung grundsätzlich ein beliebiges elektrisches Signal verstanden, welches als Reaktion auf ein vorliegendes elektrisches Signal entsteht oder erzeugt wird. Insbesondere kann es sich bei dem elektrischen Antwortsignal um ein Stromsignal handeln, beispielsweise um einen von der Source-Elektrode zu der Drain-Elektrode durch den Source-Drain-Kanal fließenden Strom. Wie bereits beschrieben, kann das elektrische Primärsignal, beispielsweise in Form des lonenstroms, das an dem Source-Drain-Kanal anliegende elektrische Potential oder die an dem Source-Drain-Kanal anliegende Spannung derart verändern, dass der Source-Drain-Kanal elektrisch leitend wird für das Stromsignal zwischen der Source-Elektrode und der Drain-Elektrode. Bei einer geeigneten zwischen der Source-Elektrode und der Drain-Elektrode anliegenden Spannung, kann das elektrische Primärsignal des *in vitro* generierten Herzschrittmachergewebes umfassend Herz-Schrittmacherzellen auf diese Weise ein elektrisches Antwortsignal in dem Source-Drain-Kanal erzeugen. Der Source-Drain-Kanal umfasst bevorzugt mindestens eine halbleitende Schicht. Weiter bevorzugt umfasst die halbleitende Schicht des Source-Drain-Kanals mindestens ein Material ausgewählt aus der Gruppe bestehend aus: einem Elementhalbleiter, insbesondere Silizium; einem Verbindungshalbleiter; einem organischen Halbleiter.

Die Erfindung betrifft ebenso ein frequenzadaptives Herzschrittmachersystem umfassend mindestens
- einen frequenzadaptiven Herzschrittmacher, umfassend
- mindestens eine Sensoreinheit, wobei die Sensoreinheit eingerichtet ist, um mindestens ein elektrisches Herzsignal zu detektieren;
- mindestens einen Pulsgenerator, wobei der Pulsgenerator eingerichtet ist, um mindestens einen elektrischen Schrittmacherimpuls zu erzeugen und dem Herz eines Patienten zuzuführen;
- mindestens eine Steuereinheit, wobei die Steuereinheit elektrisch verbunden ist mit der Sensoreinheit und dem Pulsgenerator;
- ein bioelektronisches System wie oben beschrieben, wobei das bioelektronische System leitfähig mit dem frequenzadaptiven Herzschrittmacher verbunden oder verbindbar ist, insbesondere mit der Steuereinheit.

Unter einer Sensoreinheit wird im Rahmen der vorliegenden Erfindung grundsätzlich eine beliebige Vorrichtung verstanden, welches eingerichtet ist, ein elektrisches Signal, insbesondere ein Spannungssignal oder ein Stromsignal, quantitativ oder qualitativ zu erfassen, zu registrieren, zu messen, zu empfangen, aufzunehmen oder weiterzuleiten.

Unter einem Pulsgenerator wird im Rahmend der vorliegenden Erfindung grundsätzlich eine beliebige Vorrichtung verstanden, welche eingerichtet ist, mindestens ein elektrisches Signal zu erzeugen, insbesondere ein Spannungs- und/oder ein Stromsignal. Insbesondere kann der Pulsgenerator zur Erzeugung des elektrischen Impulses, beispielsweise des elektrischen Schrittmacherimpulses, ein Elektrodenpaar umfassen.

Unter einer Steuereinheit wird im Rahmen der vorliegenden Erfindung grundsätzlich eine beliebige elektronische Vorrichtung verstanden, welche eingerichtet ist, mindestens eine Funktion des frequenzadaptiven Herzschrittmachersystems oder des frequenzadaptiven Herzschrittmachers auszuführen, anzusteuern oder auszuwerten. Die Steuereinheit kann insbesondere mindestens eine Messvorrichtung und/oder mindestens eine elektrische Energiequelle aufweisen. Weiterhin kann die Steuereinheit mindestens einen Prozessor oder Schaltkreis aufweisen, welcher eine Auswertungsfunktion des von der Sensoreinheit detektierten elektrischen Herzsignals und/oder des von dem bioelektronischen System erzeugten elektrischen Antwortsignals ausüben kann. Weiterhin kann der Prozessor oder der Schaltkreis eingerichtet sein, eine Steuerungsfunktion des Pulsgenerators auszuüben.

Insbesondere kann der Pulsgenerator eingerichtet sein, um eine Abfolge von elektrischen Schrittmacherimpulsen zu erzeugen und dem Herz eines Patienten zuzuführen;
In einer Ausführungsform des frequenzadaptiven Herzschrittmachersystems ist die elektronische Schaltung des bioelektronischen Systems eingerichtet, um mindestens ein durch ein elektrisches Primärsignal des *in vitro* generierten Herzschrittmacher-gewebes umfassend Herz-Schrittmacherzellen in einem Source-Drain-Kanal eines Feldeffekttransistors des bioelektronischen Systems erzeugtes Antwortsignal an die Steuereinheit weiterzugeben.

In einer bevorzugten Ausführungsform ist die Steuereinheit eingerichtet, um einen zeitlichen Abstand zwischen mindestens zwei aufeinanderfolgenden Antwortsignalen zu messen. Weiterhin kann die Steuereinheit eingerichtet sein, um einen zeitlichen Abstand zwischen mindestens zwei aufeinanderfolgenden elektrischen Herzsignalen zu messen. Weiterhin kann die Steuereinheit eingerichtet sein, den zeitlichen Abstand zwischen den mindestens zwei aufeinanderfolgenden Antwortsignalen mit dem zeitlichen Abstand zwischen den mindestens zwei aufeinanderfolgenden elektrischen Herzsignalen zu vergleichen. Ferner kann die Steuereinheit eingerichtet sein, um mittels des Pulsgenerators mindestens einen elektrischen Schrittmacherimpuls zu erzeugen und dem Herz eines Patienten zuzuführen, wenn der zeitliche Abstand zwischen den mindestens zwei aufeinanderfolgenden elektrischen Herzsignalen den zeitlichen Abstand zwischen den mindestens zwei aufeinanderfolgenden Antwortsignalen überschreitet.

Insbesondere kann der zeitliche Abstand zwischen den mindestens zwei aufeinanderfolgenden elektrischen Herzsignalen den zeitlichen Abstand zwischen den mindestens zwei aufeinanderfolgenden Antwortsignalen überschreiten, insbesondere deutlich überschreiten. Die Steuereinheit kann daher eingerichtet sein, den zeitlichen Abstand zwischen den mindestens zwei aufeinanderfolgenden elektrischen Herzsignalen durch einen Substitutionswert zu ersetzen, wenn eine Messdauer des zeitlichen Abstands zwischen den mindestens zwei aufeinanderfolgenden elektrischen Herzsignalen einen vorgegebenen Schwellwert überschreitet, wobei der Substitutionswert den zeitlichen Abstand zwischen mindestens zwei aufeinanderfolgenden Antwortsignalen überschreitet. Insbesondere kann es sich bei dem vorgegebenen Schwellwert um den zeitlichen Abstand zwischen den mindestens zwei aufeinanderfolgenden Antwortsignalen handeln. Ferner kann die Steuereinheit eingerichtet sein, um mittels des Pulsgenerators mindestens einen elektrischen Schrittmacherimpuls zu erzeugen und dem Herz eines Patienten zuzuführen, wenn der Substitutionswert den zeitlichen Abstand zwischen den mindestens zwei aufeinanderfolgenden Antwortsignalen überschreitet.

In einer Ausführungsform des frequenzadaptiven Herzschrittmachersystems ist die Steuereinheit eingerichtet, um dem Pulsgenerator unter Berücksichtigung des mindestens einen elektrischen Herzsignals und des mindestens einen Antwortsignals den mindestens einen zeitlichen Abstand zwischen den mindestens zwei aufeinanderfolgenden elektrischen Schrittmacherimpulsen vorzugeben.

Weiterhin kann das frequenzadaptive Herzschrittmachersystem eine Energiequelle, eine Speichermedium, sowie eine externes Bauelement umfassen, wobei das externe Bauelement eingerichtet sein kann, um Daten von dem frequenzadaptiven Herzschrittmacher, insbesondere von der Steuereinheit, mittels drahtloser Übertragung zu empfangen. Ferner kann das frequenzadaptive Herzschrittmachersystem auch weitere, hier nicht genannte Elemente umfassen.

Die vorliegende Erfindung wird durch die folgenden Ausführungsformen und Kombinationen von Ausführungsformen, die sich aus den entsprechenden Rückbezügen und Verweisen ergeben, näher illustriert. Insbesondere ist dabei anzumerken, dass in jedem Fall, in dem ein Bereich von Ausführungsformen benannt ist, beispielsweise im Kontext eines Ausdrucks wie "Eine bevorzugte Ausführungsform (5), welche einer der Ausführungsformen (1) bis (4) präzisiert" jede Ausführungsform in diesem Bereich als explizit für den Fachmann offenbart gemeint ist, d.h. die Formulierung dieses Ausdrucks für den Fachmann als synonym zu "Eine bevorzugte Ausführungsform (5), welche einer der Ausführungsformen (1), (2), (3) und (4) präzisiert" zu verstehen ist.

Gemäß einer Ausführungsform (1) betrifft die Erfindung ein bioelektronisches System, umfassend mindestens
i) *in vitro* generiertes Herzschrittmachergewebe umfassend Herz-Schrittmacherzellen;
ii) eine auf einem Halbleiter-Träger aufgebrachte elektronische Schaltung umfassend mindestens einen Feldeffekttransistor;
wobei das *in vitro* generierte Herzschrittmachergewebe umfassend Herz-

Schrittmacherzellen gemäß i) auf der auf dem Halbleiter-Träger aufgebrachten elektronischen Schaltung gemäß ii) immobilisiert und in leitfähiger Kommunikation zu dem mindestens einen Feldeffekttransistor angeordnet ist.

Eine bevorzugte Ausführungsform (2), welche Ausführungsform (1) präzisiert, betrifft ein bioelektronisches System, wobei das *in vitro* generierte Herzschrittmachergewebe umfassend Herz-Schrittmacherzellen aus Myokardzellen, bevorzugt mittels Umprogrammierung, erzeugt ist.

Eine bevorzugte Ausführungsform (3), welche Ausführungsform (1) präzisiert, betrifft ein bioelektronisches System , wobei das *in vitro* generierte Herzschrittmachergewebe umfassend Herz-Schrittmacherzellen aus embryonalen Stammzellen, bevorzugt mittels direkter Programmierung, erzeugt ist.

Eine bevorzugte Ausführungsform (4), welche Ausführungsform (1) präzisiert, betrifft ein bioelektronisches System, wobei das *in vitro* generierte Herzschrittmachergewebe umfassend Herz-Schrittmacherzellen aus multipotenten oder pluripotenten Stammzellen, bevorzugt aus pluripotenten Stammzellen, bevorzugt mittels forward-Programmierung, erzeugt ist (induzierte sinuatriale Zellkörperchen (iSABs) umfassend Herz-Schrittmacherzellen).

Eine bevorzugte Ausführungsform (5), welche Ausführungsform (4) präzisiert, betrifft ein bioelektronisches System , wobei die induzierten sinuatrialen Zellkörperchen (iSABs) umfassend Herz-Schrittmacherzellen aus nicht-humanen embryonalen Stammzellen oder nicht-humanen induzierten pluripotenten Stammzellen oder humanen induzierten pluripotenten Stammzellen oder parthenogenetischen Stammzellen oder spermatogonialen Stammzellen erzeugt sind, bevorzugt mittels forward-Programmierung, vorzugsweise aus nicht-humanen embryonalen Stammzellen oder nicht-humanen induzierten pluripotenten Stammzellen oder humanen induzierten pluripotenten Stammzellen.

Eine bevorzugte Ausführungsform (5), welche eine der Ausführungsformen (1) bis (5) präzisiert, betrifft ein bioelektronisches System, wobei das *in vitro* generierte Herzschrittmachergewebe umfassend Herz-Schrittmacherzellen zumindest teilweise in elektrischem Kontakt steht mit einer Gate-Elektrode des Feldeffekttransistors.

Eine bevorzugte Ausführungsform (7), welche Ausführungsform (6) präzisiert, betrifft ein bioelektronisches System, wobei die Gate-Elektrode mindestens einen Elektrolyten umfasst, insbesondere aus einer Lösung umfassend den mindestens einen Elektrolyten besteht.

Eine bevorzugte Ausführungsform (8), welche Ausführungsform (6) oder (7) präzisiert, betrifft ein bioelektronisches System, wobei ein an der Gate-Elektrode anliegendes elektrisches Potential direkt oder indirekt durch ein von dem *in vitro* generierten Herzschrittmachergewebe umfassend Herz-Schrittmacherzellenerzeugtes elektrisches Primärsignal beeinflussbar ist.

Eine bevorzugte Ausführungsform (9), welche Ausführungsform (8) präzisiert, betrifft ein bioelektronisches System, wobei das von dem *in vitro* generierten Herzschrittmachergewebe umfassend Herz-Schrittmacherzellen erzeugte elektrische Primärsignal mindestens einen lonenstrom umfasst, insbesondere einen lonenstrom umfassend Kalium-Ionen und/oder Calcium-Ionen.

Eine bevorzugte Ausführungsform (10), welche eine der Ausführungsformen (8) oder (9) präzisiert, betrifft ein bioelektronisches System, wobei das von dem *in vitro* generierten Herzschrittmachergewebe umfassend Herz-Schrittmacherzellen erzeugte elektrische Primärsignal durch einen physiologischen Faktor einer Umgebung des *in vitro* generierten Herzschrittmachergewebes umfassend Herz-Schrittmacherzellen beeinflussbar ist.

Eine bevorzugte Ausführungsform (11), welche eine der Ausführungsformen (8) bis (10) präzisiert, betrifft ein bioelektronisches System, wobei die Gate-Elektrode in direktem oder indirektem physischen Kontakt steht mit mindestens einem Source-Drain-Kanal des Feldeffekttransistors.

Eine bevorzugte Ausführungsform (12), welche Ausführungsform (11) präzisiert, betrifft ein bioelektronisches System, wobei in dem Source-Drain-Kanal durch das elektrische Primärsignal mittels einer Beeinflussung des an der Gate-Elektrode anliegenden elektrischen Potentials ein elektrisches Antwortsignal erzeugbar ist.

Eine bevorzugte Ausführungsform (13), welche Ausführungsform (11) oder (12) präzisiert, betrifft ein bioelektronisches System, wobei der Source-Drain-Kanal mindestens eine halbleitende Schicht umfasst.

Eine bevorzugte Ausführungsform (14), welche Ausführungsform (13) präzisiert, betrifft ein bioelektronisches System, wobei die halbleitende Schicht des Source-Drain-Kanals mindestens ein Material umfasst ausgewählt aus der Gruppe bestehend aus: einem Elementhalbleiter, insbesondere Silizium; einem Verbindungshalbleiter; einem organischen Halbleiter.

Eine Ausführungsform (15) der Erfindung betrifft ein frequenzadaptives Herzschrittmachersystem umfassend mindestens
- einen frequenzadaptiven Herzschrittmacher, umfassend mindestens eine Sensoreinheit, wobei die Sensoreinheit eingerichtet ist, um mindestens ein elektrisches Herzsignal zu detektieren;
- mindestens einen Pulsgenerator, wobei der Pulsgenerator eingerichtet ist, um mindestens einen elektrischen Schrittmacherimpuls zu erzeugen und dem Herz eines Patienten zuzuführen;
- mindestens eine Steuereinheit, wobei die Steuereinheit elektrisch verbunden ist mit der Sensoreinheit und dem Pulsgenerator;
- ein bioelektronisches System gemäß einer der Ausführungsformen (1) bis (14), wobei das bioelektronische System leitfähig mit dem frequenzadaptiven Herzschrittmacher verbunden oder verbindbar ist, insbesondere mit der Steuereinheit.

Eine bevorzugte Ausführungsform (16), welche Ausführungsform (15) präzisiert, betrifft ein frequenzadaptives Herzschrittmachersystem, wobei die elektronische Schaltung des bioelektronischen Systems eingerichtet ist, um mindestens ein durch ein elektrisches Primärsignal des *in vitro* generierten Herzschrittmacher-gewebes umfassend Herz-Schrittmacherzellen in einem Source-Drain-Kanal eines Feldeffekttransistors des bioelektronischen Systems erzeugtes Antwortsignal an die Steuereinheit weiterzugeben.

Eine bevorzugte Ausführungsform (17), welche Ausführungsform (16) präzisiert, betrifft ein frequenzadaptives Herzschrittmachersystem, wobei die Steuereinheit eingerichtet ist, um dem Pulsgenerator unter Berücksichtigung des mindestens einen elektrischen Herzsignals und des mindestens einen Antwortsignals mindestens einen zeitlichen Abstand zwischen mindestens zwei aufeinanderfolgenden elektrischen Schrittmacherimpulsen vorzugeben.

### Angeführte Literatur

Peter Fromherz, Forschungsbericht 2009 des Max-Planck-Instituts für Biochemie
WO 2015/091157 A1
WO 2017/108895 A1
WO 2013/070952 A1
US 2003/0036773 A1
US 2009/053180 A1

## Patentansprüche

1. Bioelektronisches System, umfassend mindestens
i) *in vitro* generiertes Herzschrittmachergewebe umfassend Herz-Schrittmacherzellen;
ii) eine auf einem Halbleiter-Träger aufgebrachte elektronische Schaltung umfassend mindestens einen Feldeffekttransistor;
**dadurch gekennzeichnet, dass**
das *in vitro* generierte Herzschrittmachergewebe umfassend Herz-Schrittmacherzellen gemäß i) auf der auf dem Halbleiter-Träger aufgebrachten elektronischen Schaltung gemäß ii) immobilisiert und in leitfähiger Kommunikation zu dem mindestens einen Feldeffekttransistor angeordnet ist.

2. Bioelektronisches System nach Anspruch 1, wobei das *in vitro* generierte Herzschrittmachergewebe umfassend Herz-Schrittmacherzellen aus Myokardzellen, bevorzugt mittels Umprogrammierung, erzeugt ist.

3. Bioelektronisches System nach Anspruch 1, wobei das *in vitro* generierte Herzschrittmachergewebe umfassend Herz-Schrittmacherzellen aus multipotenten oder pluripotenten Stammzellen erzeugt ist (induzierte sinuatriale Zellkörperchen (iSABs) umfassend Herz-Schrittmacherzellen).

4. Bioelektronisches System nach einem der Ansprüche 1 bis 3, wobei das *in vitro* generierte Herzschrittmachergewebe umfassend Herz-Schrittmacherzellen zumindest teilweise in elektrischem Kontakt steht mit einer Gate-Elektrode des Feldeffekttransistors.

5. Bioelektronisches System nach Anspruch 4, wobei ein an der Gate-Elektrode anliegendes elektrisches Potential direkt oder indirekt durch ein von dem *in vitro* generierten Herzschrittmachergewebe umfassend Herz-Schrittmacherzellen erzeugtes elektrisches Primärsignal beeinflussbar ist.

6. Bioelektronisches System nach Anspruch 5, wobei das von dem *in vitro* generierten Herzschrittmachergewebe umfassend Herz-Schrittmacherzellen erzeugte elektrische Primärsignal durch einen physiologischen Faktor einer Umgebung des *in vitro* generierten Herzschrittmachergewebes umfassend Herz-Schrittmacherzellen beeinflussbar ist.

7. Bioelektronisches System nach Anspruch 5 oder 6, wobei die Gate-Elektrode in direktem oder indirektem physischen Kontakt steht mit mindestens einem Source-Drain-Kanal des Feldeffekttransistors, wobei in dem Source-Drain-Kanal durch das elektrische Primärsignal mittels einer Beeinflussung des an der Gate-Elektrode anliegenden elektrischen Potentials ein elektrisches Antwortsignal erzeugbar ist.

8. Bioelektronisches System nach Anspruch 7, wobei der Source-Drain-Kanal mindestens eine halbleitende Schicht umfasst, wobei die halbleitende Schicht des Source-Drain-Kanals mindestens ein Material umfasst ausgewählt aus der Gruppe bestehend aus:
einem Elementhalbleiter; einem Verbindungshalbleiter; und einem organischen Halbleiter.

9. Frequenzadaptives Herzschrittmachersystem umfassend mindestens
- einen frequenzadaptiven Herzschrittmacher, umfassend
- mindestens eine Sensoreinheit, wobei die Sensoreinheit eingerichtet ist, um mindestens ein elektrisches Herzsignal zu detektieren;
- mindestens einen Pulsgenerator, wobei der Pulsgenerator eingerichtet ist, um mindestens einen elektrischen Schrittmacherimpuls zu erzeugen und dem Herz eines Patienten zuzuführen;
- mindestens eine Steuereinheit, wobei die Steuereinheit elektrisch verbunden ist mit der Sensoreinheit und dem Pulsgenerator;
- ein bioelektronisches System gemäß einem der Ansprüche 1 bis 8, wobei das bioelektronische System leitfähig mit dem frequenzadaptiven Herzschrittmacher verbunden oder verbindbar ist.

10. Frequenzadaptives Herzschrittmachersystem nach Anspruch 9, wobei die elektronische Schaltung des bioelektronischen Systems eingerichtet ist, um mindestens ein durch ein elektrisches Primärsignal des *in vitro* generierten Herzschrittmacher-gewebes umfassend Herz-Schrittmacherzellen in einem Source-Drain-Kanal eines Feldeffekttransistors des bioelektronischen Systems erzeugtes Antwortsignal an die Steuereinheit weiterzugeben.

## Claims

1. Bioelectronic system, comprising at least
i) *in vitro* generated cardiac pacemaker tissue comprising cardiac pacemaker cells;
ii) an electronic circuit applied to a semiconductor carrier and comprising at least one field-effect transistor;
**characterized in that**
the *in vitro* generated cardiac pacemaker tissue comprising cardiac pacemaker cells according to i) is immobilized on the electronic circuit applied to the semiconductor carrier according to ii) and is arranged in conductive communication with the at least one field-effect transistor.

2. Bioelectronic system according to Claim 1, wherein the *in vitro* generated cardiac pacemaker tissue comprising cardiac pacemaker cells is produced from myocardial cells, preferably by means of reprogramming.

3. Bioelectronic system according to Claim 1, wherein the *in vitro* generated cardiac pacemaker tissue comprising cardiac pacemaker cells is generated from multipotent or pluripotent stem cells (induced sinoatrial cell bodies (iSABs) comprising cardiac pacemaker cells).

4. Bioelectronic system according to one of Claims 1 to 3, wherein the *in vitro* generated cardiac pacemaker tissue comprising cardiac pacemaker cells is at least partially in electrical contact with a gate electrode of the field-effect transistor.

5. Bioelectronic system according to Claim 4, wherein an electrical potential applied to the gate electrode can be influenced directly or indirectly by an electrical primary signal generated by the *in vitro* generated cardiac pacemaker tissue comprising cardiac pacemaker cells.

6. Bioelectronic system according to Claim 5, wherein the electrical primary signal generated by the *in vitro* generated cardiac pacemaker tissue comprising cardiac pacemaker cells can be influenced by a physiological factor of a vicinity of the *in vitro* generated cardiac pacemaker tissue comprising cardiac pacemaker cells.

7. Bioelectronic system according to Claim 5 or 6, wherein the gate electrode is in direct or indirect physical contact with at least one source-drain channel of the field-effect transistor, wherein an electrical response signal can be generated in the source-drain channel by the electrical primary signal by means of influencing the electrical potential applied to the gate electrode.

8. Bioelectronic system according to Claim 7, wherein the source-drain channel comprises at least one semiconducting layer, wherein the semiconducting layer of the source-drain channel comprises at least one material selected from the group consisting of: an element semiconductor; a compound semiconductor; and an organic semiconductor.

9. Rate-adaptive cardiac pacemaker system comprising at least
- a rate-adaptive cardiac pacemaker, comprising
- at least one sensor unit, wherein the sensor unit is set up to detect at least one electrical cardiac signal;
- at least one pulse generator, wherein the pulse generator is set up to generate at least one electrical pacemaker pulse and to deliver it to the heart of a patient;
- at least one control unit, wherein the control unit is electrically connected to the sensor unit and the pulse generator;
- a bioelectronic system according to one of Claims 1 to 8, wherein the bioelectronic system is conductively connected or connectable to the rate-adaptive cardiac pacemaker.

10. Rate-adaptive cardiac pacemaker system according to Claim 9, wherein the electronic circuit of the bioelectronic system is set up to pass on to the control unit at least one response signal generated by an electrical primary signal of the *in vitro* generated cardiac pacemaker tissue comprising cardiac pacemaker cells in a source-drain channel of a field-effect transistor of the bioelectronic system.

## Revendications

1. Système bioélectronique comprenant au moins
i) un tissu de stimulation cardiaque généré *in vitro* et comprenant des cellules de stimulation cardiaque ;
ii) un circuit électronique appliqué sur un support semi-conducteur et comprenant au moins un transistor à effet de champ ;
**caractérisé en ce que**
le tissu de stimulation cardiaque, généré *in vitro* et comprenant des cellules de stimulation cardiaque, selon i) est immobilisé sur le circuit électronique appliqué sur le support semi-conducteur selon ii) et est disposé en communication conductrice avec l'au moins un transistor à effet de champ.

2. Système bioélectronique selon la revendication 1, le tissu de stimulation cardiaque, généré *in vitro* et comprenant des cellules de stimulation cardiaque, étant généré à partir de cellules myocardiques, de préférence par reprogrammation.

3. Système bioélectronique selon la revendication 1, le tissu de stimulation cardiaque, généré *in vitro* et comprenant des cellules de stimulation cardiaque, étant généré à partir de cellules souches multipotentes ou pluripotentes (corps cellulaires sino-auriculaires induits (iSABs) comprenant des cellules de stimulation cardiaque).

4. Système bioélectronique selon l'une des revendications 1 à 3, le tissu de stimulation cardiaque, généré *in vitro* et comprenant des cellules de stimulation cardiaque, étant au moins partiellement en contact électrique avec une électrode de grille du transistor à effet de champ.

5. Système bioélectronique selon la revendication 4, un potentiel électrique appliqué à l'électrode de grille pouvant être influencé directement ou indirectement par un signal électrique primaire généré par le tissu de stimulation cardiaque généré *in vitro* et comprenant des cellules de stimulation cardiaque.

6. Système bioélectronique selon la revendication 5, le signal électrique primaire, généré par le tissu de stimulation cardiaque généré *in vitro* et comprenant des cellules de stimulation cardiaque, pouvant être influencé par un facteur physiologique d'un environnement du tissu de stimulation cardiaque généré *in vitro* et comprenant des cellules de stimulation cardiaque.

7. Système bioélectronique selon la revendication 5 ou 6, l'électrode de grille étant en contact physique direct ou indirect avec au moins un canal source-drain du transistor à effet de champ, un signal de réponse électrique pouvant être généré dans le canal source-drain par le signal électrique primaire influant sur le potentiel électrique présent sur l'électrode de grille.

8. Système bioélectronique selon la revendication 7, le canal source-drain comprenant au moins une couche semi-conductrice, la couche semi-conductrice du canal source-drain comprenant au moins une matière choisie dans le groupe comprenant : un semi-conducteur élémentaire ; un semi-conducteur composé ; et un semi-conducteur organique.

9. Système de stimulation cardiaque adaptatif en fréquence comprenant au moins
- un stimulateur cardiaque adaptatif en fréquence, comprenant
- au moins une unité de détection, l'unité de détection étant conçue pour détecter au moins un signal cardiaque électrique ;
- au moins un générateur d'impulsions, le générateur d'impulsions étant conçu pour générer au moins une impulsion électrique de stimulation cardiaque et pour amener celle-ci au cœur d'un patient ;
- au moins une unité de commande, l'unité de commande étant reliée électriquement à l'unité de détection et au générateur d'impulsions ;
- système bioélectronique selon l'une quelconque des revendications 1 à 8, le système bioélectronique étant relié ou pouvant être relié de manière conductrice au stimulateur cardiaque adaptatif en fréquence.

10. Système de stimulation cardiaque adaptatif en fréquence selon la revendication 9, le circuit électronique du système bioélectronique étant conçu pour transmettre à l'unité de commande au moins un signal de réponse généré par un signal électrique primaire du tissu de stimulation cardiaque, généré *in vitro* et comprenant des cellules de stimulation cardiaque, dans un canal source-drain d'un transistor à effet de champ du système bioélectronique.
